(19) Europäisches Patentamt
European Patent Office
Office européen des brevets

(11) Publication number: 0 219 156 B1

(12) EUROPEAN PATENT SPECIFICATION

(45) Date of publication of patent specification: 28.11.90

(51) Int. Cl.$^5$: A 61 K 35/78 // (A61K35/78, 31:73)

(21) Application number: 86201638.3

(22) Date of filing: 22.09.86

(54) **Appetite moderating and anti-gastritis composition.**

(30) Priority: 26.09.85 IT 2228485

(43) Date of publication of application:
22.04.87 Bulletin 87/17

(45) Publication of the grant of the patent:
28.11.90 Bulletin 90/48

(84) Designated Contracting States:
AT BE CH DE FR GB IT LI LU NL SE

(56) References cited:
FR-M- 3 394
US-A-4 223 023

(73) Proprietor: Crinos Industria Farmacobiologica S.p.A.
Piazza XX Settembre, n. 2
I-22079 Villa Guardia Como (IT)

(72) Inventor: Ferro, Antonio
Via Maraini 7
CH-6900 Lugano (CH)

(74) Representative: Dragotti, Gianfranco et al
SAIC BREVETTI s.a.s. Viale Bianca Maria, 15
I-20122 Milano (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 219 156 B1

Courier Press, Leamington Spa, England.

## Description

The present invention relates to an orally administerable composition mainly having appetite moderating and antigastritis activity.

It is known that obesity and hyperphagia are a social problem besides being a therapeutical question. It is also known that the diets known and used to date are affected by the main drawback for the patient of the constant appetite or hunger sensation by which obviously the patient is disturbed in all his daily activities.

It is also known that often people suffering from disphagia are subjected to sensations of pyrosis or gastric burning and to the sensation of acidity when fasting.

The main purpose of the present invention is that of providing an orally adminsterable composition by which the problems mentioned above are essentially and advantageously solved.

This purpose is achieved by means of a composition to be orally administered and having appetite moderating and antigastritis activity, characterized by comprising an aminopolysaccharide and a soy saponin, said aminopolysaccharide being selected among chitin, chitosan and their pharmaceutically and dietetically acceptable salt, the content of said aminopolysaccharide being at least 350 mg and that of said soy saponin being at least 5 mg for a unitary dosage composition.

The influence of the nutrition of chitin and chitosan is known, the latter being extracted from some fungi and yeasts (US—B—4,363,801, Dec. 14, 1982) or prepared by direct deacetylation of the chitin, which forms the hexaskeleton of arthropodes, namely the mechanical supporting tissue of the body structure of this animal class.

Chitin and chitosan are biopolymers similar to cellulose and may be compared as regards the structure and the properties to vegetable bran, but with respect to which they possess some advantages deriving from their polycationic structure (Dietrich Knorr "Functional Properties of Chitin and Chitosan" Journal of Food Science, *47*, 593—595, 1982).

In the above mentioned US Patent some examples of salts of chitosan with acetic, citric, formic and tartaric acid, as well as with diluted mineral acids are cited.

A number of chitosan salts are known and described in the literature and generally show good solubility in water.

As the vegetable cellulose cannot be degraded by the gastroenteric juices of human beings, before their expulsion with the feces, they have some effects of relevant interest directly on the assimilation and indirectly on the haematic lipidic situation.

From the chemical point of view both chitin and chitosan consist of polymerized DL-glucosamine, insoluble in water and practically in all the commonly known organic solvents. The solubility chitosan in acidic water is owing to the possibility of salifying the free —$NH_2$ groups.

The main activity of these aminopolysaccharides in the capability of complexing the lipids with bonds which can be attributed to the density of their positive charges, and owing to their indigestibility the lipid absorption is reduced.

This property permits remarkable results to be obtained in the treatment of hyperlipemia and of hypercholesterolemia.

However a not negligible problem related to the administration of chitin or of chitosan is that of inducing a condition of constipation with self evident drawbacks and thus imposing a time limitation of the treatment or the recourse to auxiliary drugs.

Relevant interest has been also raised by the effects of the soy saponins on some particularly important, biological parameters. These effects, found both in the animal and in the human being, are particularly interesting in view of the negligible toxicity of the soy saponins, belonging to a class of substances generally known as toxic (haemolytic) (H. Ohminami, T. Hayashi, Y. Kimura, E. Okuda and S. Arichi, "Effect of Soyasaponins on Hemolysis and Acute Toxicity", 2nd Department of Medical Biochemistry, School of Medicine, Ehime University, The Research Institute of Oriental Medicine, Kinki University). Soy contains several types of saponins having the following structure:

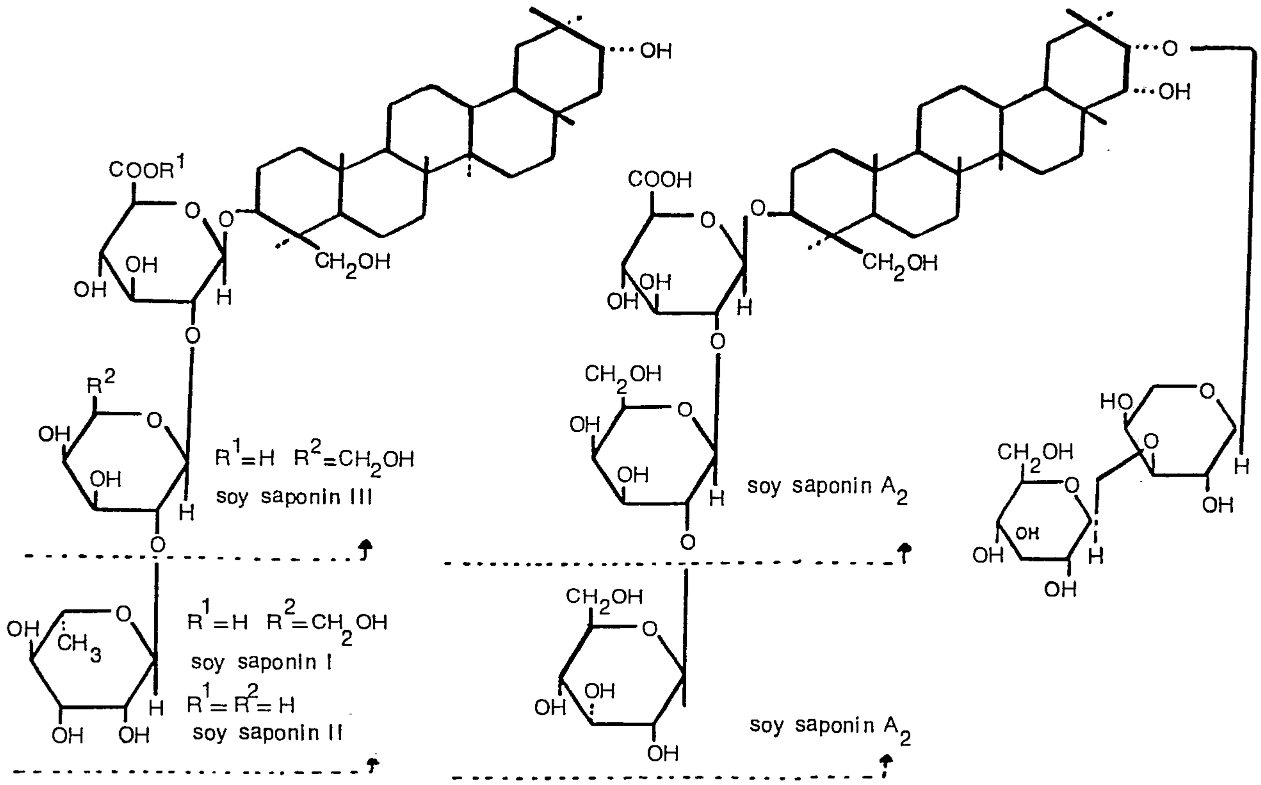

$R^1$=H $R^2$=$CH_2OH$ soy saponin III

$R^1$=H $R^2$=$CH_2OH$ soy saponin I

$R^1$=$R^2$= H soy saponin II

soy saponin $A_2$

soy saponin $A_2$

Soy saponins are essentially endowed with the following activities:

lipoperoxidasic (by removing the residues of lipoperoxides which are known for damaging the tissues).

hepatoprotective.

platletantiaggregating

antilipemic.

Soy saponins too show a very remarkable drawback, namely that of very easily inducing diarrhoea.

Lastly a further problem related to the known and proposed uses of both the said active substances is that both for chitin and chitosan and for soy saponins the daily dosages sufficient for producing satisfactory results are very high with consequent discomfort for the patients. According to the invention it has been surprisingly found that by associating the said aminopolysaccharides and the soy saponins together the same activities occur at lower dosages, moreover the composition shows novel properties of relevant interest, whereas some secondary effects, such as the compaction induced by chitosan and chitin and the diarrhoea sometimes attributable to the soy saponins, are wholly done away with.

The previously known daily dosages of chitin and chitosan to get the desired effects were of between 10 and 25 grams. For the soy saponins the doages were of between 100 and 200 milligrams.

By combining together the said two substances in a proper ratio, the dosages of the single ingredients can be reduced by 5 to 10 times, whereby the administration dosage of the aminopolysaccharide is of between 2 and 5 grams and that of the soy saponins is of between 5 and 80 milligrams. The resulting advantages are evident: as a matter of fact while it is difficult to daily ingest 10, 20 or more grams of a drug, the administration of reduced amounts in form of tablets or in other form is much easier and practicable.

The thus resulting advantages seem to be attributable to a greater mutual availability of the two active ingredients: in fact the soy saponins ahve surface activating properties, whereby the biopolymers are more readily dispersed and particularly chitosan does more readily form gels with the gastric liquids.

In vitro the gelification rate of chitosan with gastric juice is about 3 times higher if also soy saponins are present, and moreover the thus formed gel is more dense and more viscous, the concentration being the same.

It has been furthermore observed that the oily emulsions formed with saponins are more stable in the presence of chitin or chitosan, not only because of the thickening of the solvent but mainly because the emulsifying action of the soy saponins is enhanced by the aminopolysaccharides.

The association of chitin or chitosan with soy saponins is endowed with the following activities:

antihyperlipemic

antichloesterolemic

lipoperoxidasic

hepatoprotective

limiting the liquid absorption

and moreover

appetite moderating

antigastritic.

The latter two activities are characterizing the association and were never described before for the single ingredients.

Without any secondary effect, such as compaction or diarrhoea, and without any sign of intolerability even in the case of extended treatments, the association between aminopolysaccharides and saponins owing to its peculiar lipid complexing property, can be used as a slimming composition the calories ingested being the same: as a matter of fact owing to the capability of complexing and rendering undigestable part of the food lipids, the calories ingested in alimentary form are made unavailable for the organism.

Such an association shows all the advantage of the dietetic vegetable fibers and also under the chemical point of view can be defined as an animal bran with all the advantages of the true bran and moreover with the property of complexing the fats, which is typical of aminopolysaccharidic structures made surface active.

By having recourse to suitable dosages a negative caloric balance can be obtained in the case of obesity and hyperphagia. Moreover the appetite moderating effect does also eliminate the discomfort resulting from the typical hunger sensation. The gastroprotective action does not furthermore prevent pyrosis and burning from being sensed or the acid sensation (fasted), often reported by patients suffering from disphagia and having a disorderly alimentation, from occurring.

Examples of formulations

Example 1 Tablets

| | |
|---|---|
| Chitosan | mg 700 |
| soy saponins | mg 16 |
| lactose | mg 25 |
| magnesium stearate | mg 10 |

Example 2 Tablets

| | |
|---|---|
| Chitin | mg 600 |
| soy saponins | mg 10 |
| lactose | mg 50 |
| stearic acid | mg 6 |

Example 3 Tablets

| | |
|---|---|
| chitosan | mg 550 |
| soy saponins | mg 10 |
| bicalcium phosphate | mg 100 |
| polyglycol 6000 | mg 20 |

Example 4 Capsules

| | |
|---|---|
| Chitosan | mg 350 |
| soy saponins | mg 5 |
| talc | mg 15 |

Example 5 Capsules

| | |
|---|---|
| Chitin | mg 400 |
| soy saponins | mg 15 |
| starch | mg 20 |

Example 6 Oral gel

| | |
|---|---|
| Chitosan | 10.0% |
| soy saponins | 0.5% |
| citric acid | 10.0% |
| preserving and flavoring additives | enough |
| water | up to 100% |

Example 7 Oral gel

| | |
|---|---|
| Chitosan | 12.0% |
| soy saponins | 0.2% |
| hydrogen chloride | 5.0% |
| preserving and flavoring additives | enough |
| water | up to 100% |

Example 8 Oral gel

| | |
|---|---|
| chitosan | 8.0% |
| soy saponins | 0.3% |
| maleic acid | 7.0% |
| ethanol | 15.0% |
| preserving and flavoring additives | enough |
| water | up to 100% |

In the above description reference has been always made to chitin and to chitosan, the possibility being however mentioned of using their pharmaceutically and dietetically acceptable salts. It is meant that in this case the dosages shall take it into account the different molecular weight, or in other words, shall have to be referred to the respective active compound.

Lastly it is to be pointed out that by soy saponins in the above examples the commercially available products are referred to which are normally mixtures of the several soy saponins.

**Claims**

1. Oral composition for dietetic and pharmaceutical use, characterized by consisting of an aminopolysaccharide and of a soy saponin, said aminopolysaccharide being selected among chitin, chitosan and their pharmaceutically acceptable salts, the content of said aminopolysaccharide being at least 350 mg and that of said soy saponin being at least 5 mg for a unitary dosage composition.

2. Composition according to claim 1 having antigastritis and appetite moderating activity.

3. Composition according to claim 2, characterized in that the daily dosage of said aminopolysaccharide is of between 2 and 5 g and that of said soy saponin is of between 5 and 8 mg.

4. Composition according to claim 1 characterized by being in form of tablets or capsules comprising the usual excipients and vehicles, wherein the content of chitin or chitosan is of between 350 and 700 mg and that of soy saponin is of between 5 and 16 mg.

5. Composition according to claim 1, characterized in that it is in form of a gel for oral use comprising the usual excipients and vehicles, the content of chitin or chitosan being of between 8 and 12% by weight and that of soy saponin being of between 0.2 and 0.5 by weight.

**Patentansprüche**

1. Einzunehmende Zusammensetzung zum dietetischen und pharmazeutischen Gebrauch, dadurch gekennzeichnet, daß sie aus Aminopolysaccharide und Sojasaponine besteht, wobei besagte Aminopolysaccharide zwischen Chitin, Chitosan und deren pharmazeutisch akzeptierbaren Salzen selektioniert ist und wobei der Gehalt besagter Aminopolysaccharidenseife wenigstens 350 mg und jener der Sojasaponine wenigstens 5 mg pro Einheitsdosierung beträgt.

2. Zusammensetzung gemäß Anspruch 1 mit antigastritischer und appetitmildernder Tätigkeit.

3. Zusammensetzung gemäß Anspruch 2, dadurch gekennzeichnet, daß die tägliche Dosierung besagter Aminopolysaccharide zwischen 2 g und 5 g und diejenige besagter Sojasaponine zwischen 5 mg und 8 mg liegt.

4. Zusammensetzung gemäß Anspruch 1, dadurch gekennzeichnet, daß sie die Form von Tabletten oder Kapseln aufweist, einschließlich der üblichen Mittel, wobei der Anteil an Chitin oder Chitosan zwischen 350 mg und 700 mg und dergenige an Sojasaponine zwischen 5 mg und 16 mg liegt.

5. Zusammensetzung gemäß Anspruch 1, dadurch gekennzeichnet, daß sie die Form einer einzunehmenden Gelatine aufweist, einschließlich der üblichen Mittel, wobei der Anteil an Chitin oder Chitosan zwischen 8 und 12 und derjenige an Sojasaponine von zwischen 0.2 und 0.5 Gewichtsprozenten liegt.

**Revendications**

1. Composition orale pour usage diététique et pharmaceutique caractérisé par le fiat qu'elle consiste d'une aminopolysaccharide et d'une saponine de soja, ladite aminopolysaccharide étant sélectionée parmi chitin, chitosan et leurs sels pharmaceutiquement acceptables, le contenu de ladite aminopolysaccharide étant au moins de 350 mg et celui de ladite saponine de soja étant au moins de 5 mg par dosage unitaire.

2. Composition selon la revendication 2, caractérisée par le fait qu'elle possède un activité antigastrique et modérant l'appétit.

3. Composition selon la revendication 2, caractérisée par le fait qu le doage journalier de ladite aminopolysaccharide est entre 2 et 5 g et celui de ladite saponine de soja est entre 5 et 8 mg.

4. Composition selon la revendication 1, caractérisée par le fait qu'elle présente la forme de cachets ou capsules en comprenant les véhicules abituels, où le contenu de chitin ou de chitosan est entre 350 et 700 mg et celui de saponine de soja est entre 5 et 16 mg.

5. Composition selon la revendication 1, caractérisée par le fait qu'elle existe en forme de gelée pour usage oral en comprenant les véhicules abituels, le contenu de chitin ou de chitosan étant entre 8 e 12% par poids et celui de saponine de soja étant entre 0.2 et 0.5% pa poids.